Europäisches Patentamt

European Patent Office

Office européen des brevets

(19)

(11) Numéro de publication: **0 018 905**
**B1**

(12) **FASCICULE DE BREVET EUROPEEN**

(45) Date de publication du fascicule du brevet: **02.03.83**

(21) Numéro de dépôt: **80400579.1**

(22) Date de dépôt: **25.04.80**

(51) Int. Cl.³: **G 01 N 33/48,**
**G 01 N 11/16**

(54) **Dispositif de mesure de paramètres de coagulation ou de lyse.**

(30) Priorité: **27.04.79 FR 7910915**

(43) Date de publication de la demande:
**12.11.80 Bulletin 80/23**

(45) Mention de la délivrance du brevet:
**02.03.83 Bulletin 83/9**

(84) Etats contractants désignés:
**BE DE GB IT NL**

(56) Documents cités:
**FR - A - 2 370 971**
**FR - A - 2 389 137**
**US - A - 2 550 052**

(73) Titulaire: **PROBIO DMS**
**Centre Commercial de Fouilleuse rue des**
**Mazurières**
**F-92500 Rueil Malmaison (FR)**

(72) Inventeur: **Do Mau, Tung**
**28 bis, rue Louis Hubert**
**F-78140 Velizy (FR)**
Inventeur: **Do Mau, Lam**
**28 bis, rue Louis Hubert**
**F-78140 Velizy (FR)**

(74) Mandataire: **Fort, Jacques et al,**
**CABINET PLASSERAUD 84, rue d'Amsterdam**
**F-75009 Paris (FR)**

Courier Press, Leamington Spa, England.

# Dispositif de mesure de paramétres de coagulation ou de lyse

L'invention a pour objet un dispositif de mesure ou d'observation de paramètres représentatifs de la coagulation ou de la lyse d'un liquide coagulable, du type comprenant un organe de torsion constitué par un fil suspendu sensiblement verticalement, une masse d'entraînement supportée par cet organe de torsion et destinée à être plongée dans ledit liquids, des moyens pour conférer, à un récipient contenant ce liquide, des oscillations d'amplitude et de période déterminées autour d'un axe sensiblement confondu avec celui de l'organe de torsion et des moyens de détection de l'amplitude de la torsion dudit organe, ces moyens de détection comprenant un élément inducteur déplaçable en fonction de la torsion dudit organe, des moyens détecteurs de position fixes, à induction, propres à coopérer avec l'élément inducteur déplaçable de façon que, sur leur borne de sortie, apparaisse un signal électrique représentatif de l'amplitude des oscillations dudit organe de torsion. Un tel dispositif est connu du brevet FR—A 2370971.

Le dispositif décrit à titre d'exemple dans le brevet FR—A—2370971 utilise une masse d'entraînement suspendue à l'organe de torsion, constitué par un fil ou ruban. Pour que ce montage fonctionne de façon satisfaisante, il est nécessaire d'éviter toute inclinaison de la table de travail sur l'horizontale.

La présente invention vise à fournir un dispositif répondant mieux que ceux antérieurement connus aux exigences de la pratique, notamment en ce qu'il est susceptible de fonctionner de façon satisfaisante sans exiger des réglages précis d'horizontalité et un dispositif d'amortissement d'oscillations transverses.

Dans ce but, l'invention propose notamment un dispositif du type ci-dessus défini, dont le fil est tendu entre un point d'ancrage supérieur et un point d'ancrage inférieur, le fil étant fixé à un étrier qui supporte la masse en un point situé au-dessous du point d'ancrage inférieur.

Le terme "fil" doit être interprété dans un sens large et comme couvrant notamment le cas où le fil comporte plusieurs tronçons reliés par un élément rigide.

Dans un mode de réalisation préferé, la masse est fixée de façon amovible à l'extrémité inférieure d'un étrier entourant l'un des points d'ancrage (typiquement le point d'ancrage inférieur) et portant à sa partie supérieure l'élément inducteur.

L'invention vise également à améliorer la résistance du dispositif aux chocs et efforts provenent notamment du transport et des manipulations.

Dans ce but at selon un mode particulier de réalisation, l'étrier forme un equipage rotatif avec un disque centré sur le fil et susceptible d'être bloqué en place par un mécanisme de verrouillage.

L'invention propose encore d'autres perfectionnements qui apparaîtront à la lecture de la description qui suit d'un dispositif constituant un mode particulier de réalisation, donné à titre d'exemple non limitatif. La description se réfère aux dessins qui l'accompagnent dans lesquels:

la figure 1 est un exemple de thromboélastogramme, c'est-à-dire de courbes représentatives de la variation en fonction du temps de l'amplitude des oscillations de la masse d'un dispositif de mesure, à fréquence et amplitude constantes des oscillations du récipient contenant le liquide étudié,

la figure 2 est un schéma de principe en perspective montrant les organes mécaniques principaux du dispositif,

la figure 3 montre les organes mécaniques d'un dispositif correspondant au schéma de la figure 2, en coupe suivant un plan passant par le fil de suspension,

la figure 4 est un schéma en perspective, montrant le mécanisme d'entraînement du chariot du dispositif,

la figure 5 est un schéma électrique de principe, montrant l'alimentation électrique du moteur de déplacement du chariot,

la figure 6 est un synoptique de principe, montrant un mode possible de commande du dispositif par microprocesseur et de visualisation des résultats.

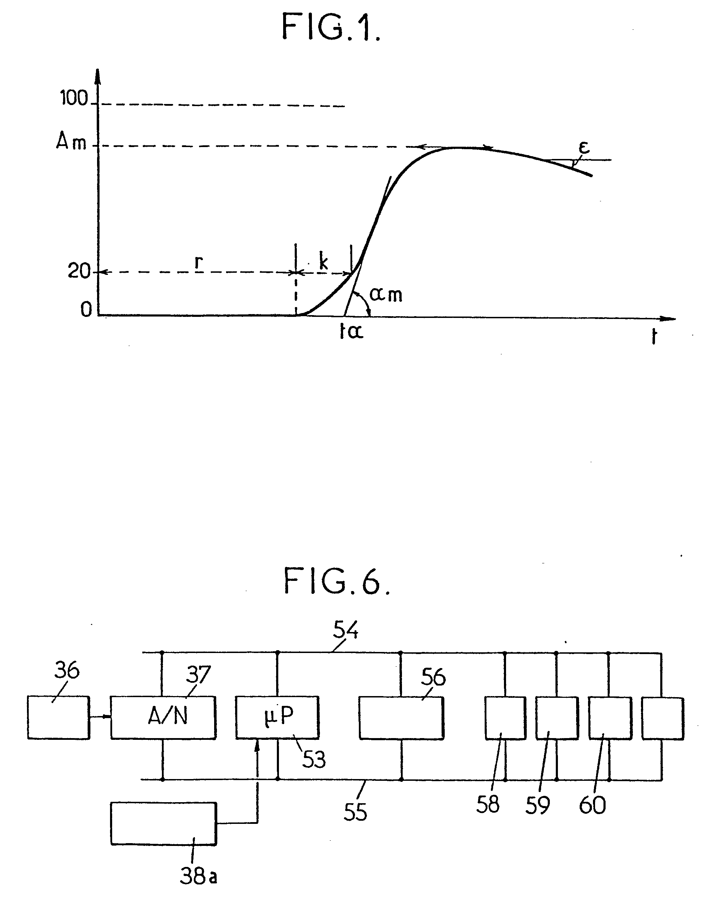

Avant de décrire l'invention, on rappellera, en faisant référence à la figure 1, les paramètres les plus importants d'exploitation d'un thrombo élastogramme.

On a indiqué sur cette figure 1:

—l'amplitude maximum Am qui peut être exprimée sous forme d'une fraction de 0 à 100% de l'amplitude angulaire d'oscillations communiquées au récipient contenant le produit à étudier,

—le temps $r$ au bout duquel on commence à détecter une oscillation de la masse plongeant dans le produit à étudier,

—le délai $k$, à partir de l'expiration de $r$, au bout duquel l'amplitude d'oscillation a atteint 20% de l'amplitude du récipient,

—la valeur maximum tangente $\alpha_m$ de la pente ascendante,

—l'abscisse t$\alpha$ correspondant à la tangente de pente maximum,

—la pente tg$\varepsilon$ de la partie décroissante du thromboélastogramme.

Dans une version élaborée de l'appareil qui sera maintenant décrite, l'ensemble de ces données peut être calculé et affiché, ce qui en simplifie considérablement l'exploitation.

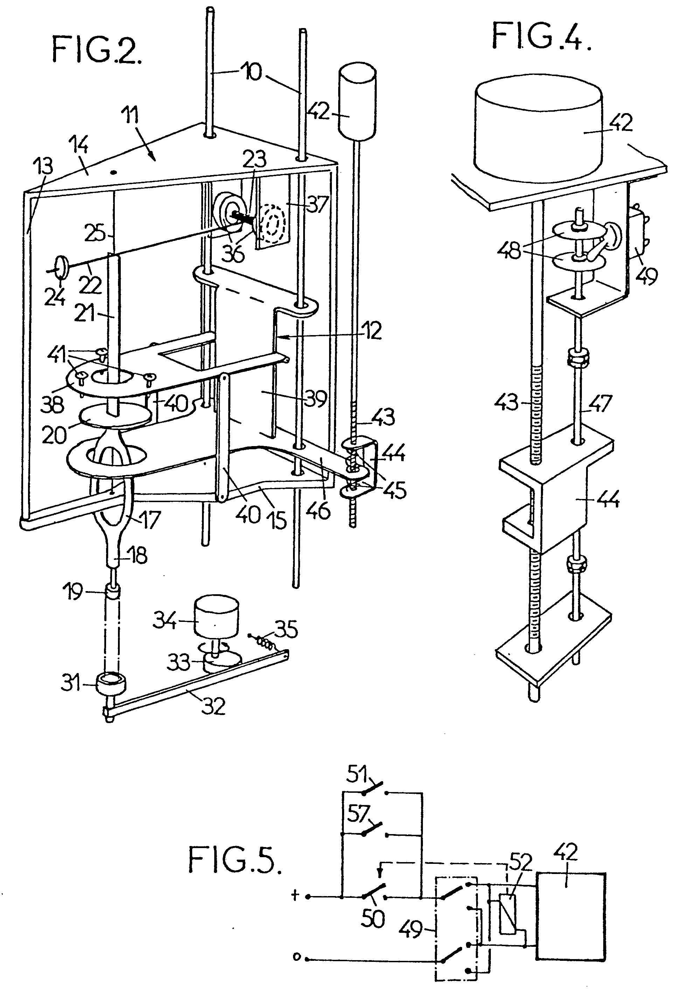

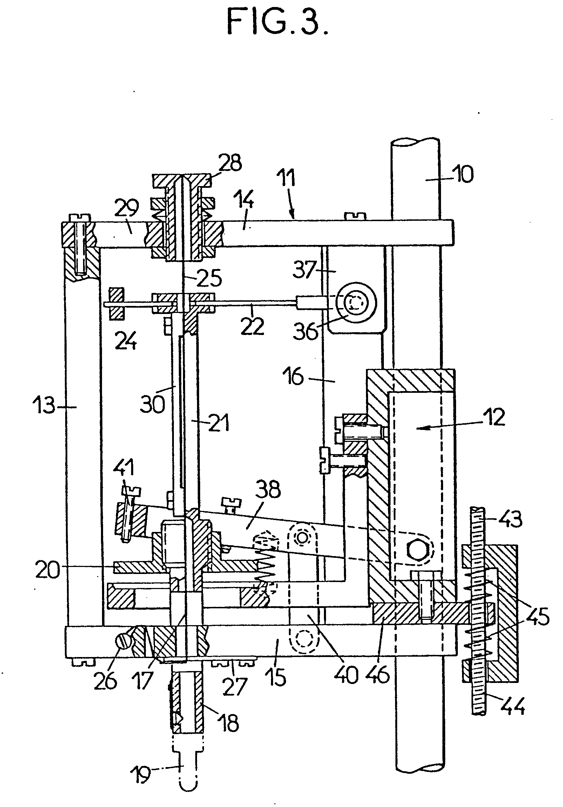

Dans son principe, le dispositif suivant l'invention comprend un bâti, non représenté, muni de deux colonnes verticales 10. Sur ces colonnes peuvent coulisser verticalement, d'une part, un châssis 11, d'autre part, un chariot 12. Le châssis 11, représenté sur la figure 2 sous forme d'un simple U dont les branches sont reliées par une tige de liaison 13, sera dans la pratique en plusieurs pièces assemblées. Dans le mode de réalisation montré en figure 3, il comporte deux plaques horizontales 14 et 15 reliées par la tige 13 et par deux chandelles symétriques 16. Les plaques 14 et 15 sont percées de trous situés en arrière des chandelles 16, de diamètre correspondant à celui des colonnes 10.

Le châssis 11 porte l'équipage oscillant du dispositif. Cet équipage comprend un étrier 17 muni, à sa partie inférieure, d'un embout 18 de fixation amovible de la masse 19 prévue pour être plongée dans le produit coagulable à étudier. Cet étrier encadre la partie terminale avant du plateau inférieur 15. Sa partie supérieure est munie d'un disque de verrouillage 20 dont le rôle sera indiqué plus loin et se prolonge par une barrette 21. A l'extrémité supérieure de cette barrette, est fixé un bras horizontal 22 portant un élément inducteur 23, généralement constitué par un noyau de ferrite, formant l'élément mobile des moyens de détection. Un contrepoids 24 réglable en position sur le bras 22 permet d'équilibrer ce dernier.

L'équipage mobile est porté par un fil 25 tendu entre deux points d'ancrage portés par les plateaux supérieur 14 et inférieur 15. On voit que, grâce à la présence de l'étrier 17, il est possible de placer la masse d'entraînement 19 au-dessous du plateau inférieur 15, donc de la mettre en place sans difficulté dans le récipient contenant le produit coagulable à étudier.

Les points d'ancrage peuvent avoir la constitution montrée en figure 3. Le fil 25 est immobilisé sur le plateau inférieur 15 par une vis 26, traverse le plateau et retraverse ce plateau, cette fois vers le haut, en un emplacement déterminé de façon précise par une plaquette de centrage 27. L'extrémité haute du fil 25 est fixée à une douille filetée 28 retenue sur le plateau 14 dans une position ajustable à l'aide de deux écrous 19 que permettent de régler la tension du fil.

De nombreux modes de fixation de l'équipage rotatif sur le fil peuvent être utilisés. Dans le mode de réalisation montré en figure 3, le fil est pincé entre la barrette 21 et une plaquette amovible 30.

Comme on l'a déjà indiqué, on pourra utiliser, au lieu d'un fil d'un seul tenant, plusieurs tronçons successifs reliés par l'équipage mobile.

Le dispositif comporte également des moyens pour conférer, à un récipient contenant le liquide coagulable à étudier, des oscillations autour d'un axe sensiblement confondu avec celui du fil 25. Ces moyens ne seront pas décrits en détail, car ils peuvent être identiques à ceux mentionnés dans le brevet cité. Sur la figure 1, ces moyens comportent un support 31 pour le récipient contenant le liquide, muni d'un bras 32 oscillant autour d'un axe vertical sous l'action d'une came 33 mise en rotation par un moteur électrique 34. Un ressort 35 maintient le bras en contact avec la came.

Le châssis 11 porte encore la partie fixe des moyens détecteurs de position, constitués dans le mode de réalisation illustré par deux bobines 36 placées sur un dé 37 fixé au plateau 14. Enfin, un moteur électrique, non représenté, peut être prévu pour descendre le châssis jusqu'au moment où la masse 19 plonge dans le récipient pour le relever.

Le dispositif comporte un mécanisme de verrouillage de l'équipage mobile, qui bloque celui-ci aussi bien en translation verticale qu'en rotation et permet à l'ensemble de subir sans dommage des chocs et manipulations. Ce mécanisme de verrouillage comporte le chariot 12 et un plateau 38. Ce plateau est monté sur un montant vertical 39 du chariot 12, de façon à pouvoir osciller autour d'un axe perpendiculaire au fil 25. Deux biellettes 40 constituent, entre le plateau 38 et le plateau inférieur 15, une liaison articulée autour de deux axes parallèles à l'axe de basculement du plateau 38 sur le montant 39. Ainsi, si l'on déplace le chariot 12 vers le haut à partir de la position où il est représenté en figure 2, le plateau 38 bascule vers le bas. Le disque 20 peut ainsi être pincé entre une palette inférieure appartenant au chariot 12 et des vis réglables 41 portées par le plateau 38. Inversement, si le chariot 12 descend par rapport au châssis 11, le plateau 38 et la palette s'écartent symétriquement du disque 20 et le libèrent.

Les déplacements du chariot 12 sont commandés par un mécanisme prévu pour éviter toute application d'efforts excessifs. Il comporte un moteur électrique réversible 42 (figures 2 et 3) dont le bâti est solidaire du châssis 11. Ce moteur, dont le sens de rotation dépend de la polarité qui lui est appliquée, entraîne une tige filetée 43 vissée dans une chape 44 immobilisée en rotation. La mise en marche du moteur provoque la montée ou la descente de la chappe 44, suivant le sens de rotation du moteur. Un dispositif de centrage élastique, constitué, dans le mode de réalisation illustré, par deux ressorts 45 (figures 2 et 3), tend à maintenir une patte 46 solidaire du chariot 12 au milieu de la chape 44. Ainsi, lorsque le chariot 12 vient en butée haute ou basse contre le châssis, l'application d'efforts excessifs au moteur 42 est évitée par la compression du ressort 45 correspondant.

Le dispositif comporte avantageusement une commande unique de montée et de descente, évitant toute fausse manoeuvre. Cela implique la présence d'un circuit qui impose au moteur, après chaque venue en butée, que son déplacement à la mise en action suivante soit inverse du précédent. Dans le mode de réalisation montré en figure 4, l'équerre 44, une tige 37 solidaire de la patte 46 (et, de plus, immobilisant en rotation l'équerre 44) portent deux rondelles 48 de commande d'un commutateur inverseur 49. Le montage de ce commutateur peut être celui indiqué en figure 5: le circuit d'alimentation du moteur 42 comporte le contact mobile 50 d'un relais non polarisé, en parallèle avec un interrupteur à commande manuelle 51, et le commutateur inverseur 49. La bobine 52 du relais est placée directement aux bornes du moteur. La fermeture de l'interrupteur 51 provoque l'alimentation du moteur avec une polarité fixée par le commutateur 49. Dès que cette alimentation est effective, le contact 50 vient au travail et on peut relâcher l'interrupteur 51, pouvant être constitué par un bouton poussoir.

Le moteur 42 entraîne le chariot jusqu'à sa position de butée, pour laquelle le commutateur 49 s'inverse. Cette inversion provoque une brève coupure qui désexcite le relais de sorte que le contact 50 s'ouvre. Une nouvelle pression sur l'interrupteur 51 provoquera le déplacement du moteur 42 en sens contraire du sens précédent.

La commande du système et les calculs peuvent être effectués par voie purement électronique, de façon à fournir à l'utilisateur des résulats immédiatement exploitables. Pour cela, on peut utiliser le montage dont un synoptique de principe est donné en figure 6, qui fonctionne en numérique. Ce montage comporte un microprocesseur 53 (par exemple SC/MP de NATIONAL SEMICONDUCTOR®, 80—82 INTEL® ou 6800 de MOTOROLA®) relié à des lignes bus 54 d'adresses et 55 de données. Des mémoires mortes supplémentaires 56 contenant les programmes d'utilisation peuvent être également reliées aux lignes 54 et 55. Le signal de sortie fourni par les moyens détecteurs de position fixes 36 est numérisé dans un convertisseur 37 et appliqué au microprocesseur, muni d'une horloge externe 38a. Le montage comporte encore un certain nombre d'organes de visualisation, dont le nombre et la fonction dépendront du nombre de paramètres que l'on veut simultanément ou successivement afficher.

Le dispositif comportera encore un certain nombre de boutons d'appel d'affichage de certains paramètres. Le programme devra permettre, à partir des données fournies par le capteur 36, de déterminer en permanence l'amplitude A sous forme numérique, et de mémoriser ceux des paramètres Am, r, k, tg, $\alpha$m, t$\alpha$ et $\varepsilon$ dont on souhaite disposer.

Un bouton de début de séquence peut être prévu, le microprocesseur assurant alors l'ensemble des opérations nécessaires, et commandant notamment le fonctionnement du moteur 42 à l'aide d'un interrupteur supplémentaire 57 (figure 5).

Dans la pratique, il sera souvent utile de prévoir plusieurs dispositifs de visualisation permettant de fournir les indications ci-après.

Une première fenêtre de visualisation 58, de grande taille, sert à afficher en permanence le temps $t$ àpartir du début de la mesure jusqu'à ce que celle-ci soit terminée. Une second fenêtre 59 affiche l'amplitude instantanée A des oscillations au cours de la mesure. Une troisième fenêtre 60 affiche le paramètre $r$ lorsqu'il est devenu disponible.

Le microprocesseur peut être programmé pour réaliser notamment la séquence d'opérations suivante, déclenchée par une pression sur un bouton unique de mise en route.

—le châssis étant initialement en position haute, le microprocesseur provoque la descente, à vitesse uniforme, jusqu'à ce que la masse soit dans la cuve de mesure, puis arrête le moteur,

—une fois la masse en place, la mesure démarre automatiquement,

—l'appareil s'arrête automatiquement lorsque le thromboélastogramme commence à présenter une pente descendante uniforme tg$\varepsilon$; le châssis se relève alors automatiquement vers sa position haute de repos et, en même temps, un signal visuel ou auditif de fin de mesure est émis: il peut s'agir de l'affichage du mot "fin" dans la fenêtre de mesure 58,

—si, pour une raison quelconque, on souhaite arrêter une mesure en cours, il suffit d'appuyer sur le bouton pour déclencher le relèvement du châssis jusqu'à la position haute de repos, tandis que le processus de mesure est remis à zéro,

—à la fin de la mesure, il peut être prévu l'affichage permanent de l'amplitude maximum Am dans la fenêtre 58 et l'apparition des autres paramètres dans les autres fenêtres, sur appel à l'aide de boutons particuliers.

La possibilité d'afficher dans une même fenêtre de visualisation 58 l'un quelconque de plusieurs paramètres pré-définis représente un facteur important d'économie et de commodité, surtout sur un appareil dont le boîtier est de petite taille.

Une solution commode pour réaliser un affichage dépourvu de toute ambiguïté consiste à prévoir, sur le boîtier de l'appareil, des boutons de commande en nombre égal à celui des paramètres à afficher dans la fenêtre 58, chaque bouton étant identifié, d'une part, par un numéro de référence et, d'autre part, par une légende en clair. L'électronique associée à la fenêtre est alors prévue pour afficher, dans la partie gauche, le numéro du bouton et donc du paramètre affiché, et, à droite, la valeur numérique de ce paramètre.

Ce mode de visualisation peut être réalisé de façon simple dans un appareil comportant un micro-processeur. Il suffit pour cela d'affecter à chacun des boutons de commande une adresse et de programmer le micro-processeur de façon qu'il interroge, à intervalles réguliers, l'état des boutons. Cette interrogation s'effectue en émettant sur le bus d'adresse un code d'appel qui est transmis à l'ensemble des boutons par une ligne d'interrogation. Si un bouton est actionné, il est identifié par un comparateur associé à ce bouton qui débloque une porte d'émission sur le bus de données d'un nombre que le calculateur interprète comme l'adresse en mémoire d'un paramètre calculé. La valeur de ce paramètre est alors émise vers le circuit de visualisation associé à la fenêtre. Les circuits de maintien peuvent être prévus de façon que le nombre reste affiché jusqu'à activation d'un autre bouton.

**Revendications**

1. Dispositif de mesure ou d'observation de paramètres représentatifs de la coagulation et/ou de la lyse d'un liquide coagulable, dispositif comprenant un organe de torsion constitué par un fil (25) suspendu sensiblement verticalement, une masse d'entraînement (19) supportée par cet organe de torsion et destinée à être plongée dans ledit liquide, des moyens (31—35) pour conférer, à un récipient contenant ce liquide, des oscillations d'amplitude et de période déterminées autour d'un axe sensiblement confondu avec celui de l'organe de torsion et des moyens de détection (22—24) de 'amplitude de la torsion dudit organe, ces moyens de détection comprenant un élément inducteur (23) déplaçable en fonction de la torsion dudit organe, des moyens détecteurs de position (36) fixes, à induction, propres à coopérer avec l'élément inducteur déplaçable de façon que, sur leur borne de sortie, apparaisse un signal électrique représentatif de l'amplitude des oscillations dudit organe de torsion, caractérisé en ce que le fil (25) est tendu entre un point d'ancrage supérieur et un point d'ancrage inférieur, le fil étant fixé à un étrier (17) qui supporte la masse (19) en un point situé au-dessous du point d'ancrage inférieur.

2. Dispositif suivant la revendication 1, caractérisé en ce que ladite masse (19) est fixée à l'extrémité inférieure de l'étrier (17) qui entoure le point d'ancrage inférieur et porte, à sa partie supérieure, ledit élément inducteur (23).

3. Dispositif suivant la revendication 2, caractérisé en ce que l'étrier (17) forme un équipage rotatif avec un disque (20) centré sur le fil (25) et susceptible d'être bloqué en place par un mécanisme de verrouillage (12, 38, 40, 41).

4. Dispositif suivant la revendication 1, 2 ou 3, caractérisé en ce qu'il comporte un châssis (11) ayant un bras supérieur (14) et un bras inférieur (15) sur lesquels sont prévus les points d'ancrage, le bras inférieur traversant l'étrier (17), ledit châssis étant avantageusement muni de moyens (42—49) permettant de le déplacer verticalement pour plonger la masse (19) dans le récipient et l'en retirer.

5. Dispositif suivant les revendications 3 et 4, caractérisé en ce que le mécanisme de verrouillage comprend un chariot (12) muni de moyens (42—46) permettant de la déplacer parallèlement au fil (25) par rapport au châssis (11), vers le disque (20) et à partir du disque, et un plateau (38) articulé sur le chariot et sur le châssis de façon que le disque soit pincé symétriquement par le chariot (12) et le plateau (38) lors du déplacement du chariot vers le disque.

6. Dispositif suivant la revendication 5, caractérisé en ce que le mécanisme de verrouillage comprend un moteur (42) d'entraînement du chariot par rapport au châssis, par l'intermédiaire d'un dispositif de centrage élastique (44, 45) de limitation d'effort.

7. Dispositif suivant la revendication 6, caractérisé en ce que le dispositif de centrage élastique (44, 45) est associé à un commutateur (48, 49) inversant la polarité d'une source d'alimentation du moteur (42) et coupant ladite alimentation lors de la venue en butée dudit dispositif de centrage élastique.

8. Dispositif suivant l'une quelconque des revendications précédentes, caractérisé en ce qu'il comporte des moyens de calcul et d'affichage (53—60), avantageusement à micro-processeur (53), permettant de calculer lesdits paramètres représentatifs, tels que notamment l'amplitude maximum des oscillations, le temps au bout duquel on commence à détecter une oscillation, la valeur maximum de la pente ascendante de la courbe représentative de l'amplitude en fonction du temps, et les pentes maximales, de les mémoriser et de les afficher.

9. Dispositif suivant la revendication 8, caractérisé en ce que les moyens d'affichage comprennent une fenêtre d'affichage (58) et des moyens sélecteurs à commande par l'opérateur, permettant de visualiser dans la fenêtre l'un quelconque des paramètres conservés en mémoire.

**Patentansprüche**

1. Vorrichtung zur Messung oder Beobachtung der Koagulations- und/oder Lyse-Parameter einer koagulierbaren Flüssigkeit mit einem Torsionsorgan, das von einem im wesentlichen senkrecht aufgehängten Faden gebildet ist, mit einer vom Torsionsorgan getragenen und in die Flüssigkeit eintauchbaren Mitnehmermasse (19), mit Mitteln (31—35) zum Übertragen von Schwingungen bestimmter Amplitude und Periode auf ein die Flüssigkeit enthaltendes Gefäß um eine Achse, die im wesentlichen mit derjenigen des Torsionsorgans zusammenfällt, und mit Mitteln (22—24) zur Bestimmung der Amplitude der Verbindung des Torsionsorgans, wobei diese Mittel ein in Abhängigkeit von der Verbindung des Torsionsorganes verschiebbares Induktorelement (23) sowie ortsfeste, induktiv wirkende Positionsfeststellmittel (36) umfassen, welche mit dem Induktorelement zusammenwirken, das seinerseits derart verschiebbar ist, daß an der Ausgangsklemme der Positionsfeststellmittel ein elektrisches, für die Schwingungsamplitude des Torsionsorgans repräsentatives Signal auftritt, dadurch gekennzeichnet, daß der Faden (25) zwischen einem oberen Verankerungspunkt und einem unteren Verankerungspunkt ausgespannt ist, und daß der Faden an einem Bügel (17) befestigt ist, der die Masse (19) an einem unterhalb des unteren Verankerungspunktes gelegenen Punkt trägt.

2. Vorrichtung nach Anspruch 1, dadurch gekennzeichnet, daß die Masse (19) am unteren Ende des Bügels (17) befestigt ist, welcher den unteren Verankerungspunkt umgreift und an seinem Oberteil das Induktorelement (23) trägt.

3. Vorrichtung nach Anspruch 2, dadurch gekennzeichnet, daß der Bügel (17) zusammen mit einer Scheibe (20) ein drehbares Organ bildet, und daß die Scheibe auf dem Faden (25) zentriert und durch einen Verriegelungsmechanismus (12, 38, 40, 41) am Platz arretierbar ist.

4. Vorrichtung nach Anspruch 1, 2 oder 3, dadurch gekennzeichnet, daß sie einen Rahmen mit einem oberen Arm (14) und einem unteren Arm (15) umfaßt, auf denen die Verankerungspunkte vorgesehen sind, wobei der untere Arm den Bügel (17) durchquert, und daß der Rahmen vorteilhafterweise mit Mitteln versehen ist (42—49), die es gestatten, ihn vertikal zu verschieben, um so die Masse (19) in das Gefäß einzutauchen und wieder herauszuziehen.

5. Vorrichtung nach Anspruch 3 und 4, dadurch gekennzeichnet, daß der Verriegelungsmechanismus einen Schlitten (12) umfaßt, der mit Mitteln (42—46) versehen ist, die es gestatten, ihn parallel zum Faden (25) mit Bezug auf den Rahmen (11) zur Scheibe (20) hin und von dieser weg zu verschieben, und daß am Schlitten und am Rahmen eine Platte (38) derart angelenkt ist, daß die Scheibe bei einer Verschiebung des Schlittens zur Scheibe hin symmetrisch durch den Schlitten (12) und die Platte (38) eingeklemmt ist.

6. Vorrichtung nach Anspruch 5, dadurch gekennzeichnet, daß der Verriegelungsmechanismus einen Antriebsmotor (42) für den Schlitten relativ zum Rahmen umfaßt, und zwar unter Zwischenschaltung einer elastischen, der Kraftbegrenzung dienenden Zentriereinrichtung (44, 45).

7. Vorrichtung nach Anspruch 6, dadurch gekennzeichnet, daß die elastische Zentriereinrichtung (44, 45) mit einem Umschalter (48, 49) verbunden ist, der die Polarität einer Speisequelle des Motors (42) umkehrt und die Speisequelle beim Anschlagen der elastischen Zentriereinrichtung abschaltet.

8. Vorrichtung nach einem der voranstehenden Ansprüche, dadurch gekennzeichnet, daß sie Rechenund Anzeigemittel (53—60), vorzugsweise einen Mikroprozessor (53) umfaßt, welche eine Berechnung, Speicherung und Anzeige der genannten Parameter ermöglichen, insbesondere der maximalen Amplitude der Schwingungen, der Zeitdauer, die bis zum Beginn einer Beobachtung von Schwingungen verstreicht, des maximalen Anstieges der die Amplitude in Abhängigkeit von der Zeit darstellenden Kurve und der maximalen Neigungen.

9. Vorrichtung nach Anspruch 8, dadurch gekennzeichnet, daß die Anzeigemittel ein Anzeigefenster (58) und durch eine Bedienungsperson steuerbare Auswahlmittel umfassen, die es gestatten, in dem Fenster irgeneinen der gespeicherten Parameter sichtbar zu machen.

**Claims**

1. A device for measuring or observing parameters representative of the coagulation and/or lysis of a coagulable liquid, comprising a torsion member consisting of a substantially vertically suspended wire (25), a driving body (19) supported by said torsion member and arranged to be dipped into said liquid, means (31—35) for communicating oscillations to a receptacle containing said liquid, said oscillations being about an axis which is substantially the same as that of the torsion member and having a predetermined amplitude and period, and means (22, 24) for detecting the amplitude of the torsion of said member, said detection means comprising an induction element so movable that, on their output, there appears an electrical signal which is representative of the amplitude of the oscillations of said torsion member, characterized in that said wire is stretched between a higher anchoring point and a lower anchoring point, said wire being fixed to a stirrup (17) which supports the body (19) at a point which is located at a lower level than the lower anchoring point.

2. Device according to claim 1, characterized in that said body (19) is secured to the lower end of the stirrup (17) which straddles the lower anchoring point and carries said induction element (23) at its upper portion.

3. Device according to claim 2, characterized in that the stirrup (17) together with a disk (20) centered on the wire (25) and adapted to be locked in place by a locking mechanism (12, 38, 40, 41).

4. Device according to claim 1, 2 or 3, characterized in that it comprises a frame (11) having a higher arm (14) and a lower arm (15) on which the anchoring points are provided, the lower arm traversing the stirrup (17), said frame being advantageously provided with means (42—49) for displacing it vertically for dipping the body (19) into the receptacle and withdraw it.

5. Device according to claims 3 and 4, characterized in that the locking mechanism comprises a carriage (12) provided with means (42, 46) for moving it parallel to the wire (25) with respect to the frame (11) toward and away from the disk (20) and a plate (38) pivotably connected to the carriage and to the frame whereby the disk is symmetrically clamped by the carriage (12) and the plate (38) when the carriage is moved toward the disk.

6. Device according to claim 5, characterized in that the locking mechanism comprises a motor (42) for driving the carriage with respect to the frame, through a force limitation resilient centering device (44, 45).

7. Device according to claim 6, characterized in that the resilient centering device (44, 45) is associated with a switch (48, 49) for inverting the polarity of a power source for the motor (42) and for cutting off said source when said resilient centering device comes into abutment.

8. Device according to anyone of the preceding claims, characterized in that it comprises computing and display means (53—60), advantageously including a microprocessor (53), for computing said representative parameters, such as particularly the maximum amplitude of the oscillations, the time period after which oscillation detection begins, the maximum value of the upward slope of the curve representing the amplitude variation as a function of time, and the maximum slopes, for storing them and for displaying them.

9. Device according to claim 8, characterized in that the display means comprises a display window (58) and operator controlled selector means for authorizing display of anyone of the parameters stored in a memory in said window.

# FIG.1.

# FIG.6.

FIG.2.

FIG.4.

FIG.5.

# FIG.3.